# FASCICULE DE BREVET EUROPEEN

(11) **EP 3 648 842 B1**
(45) Date de publication et mention de la délivrance du brevet: **11.08.2021**
(21) Numéro de dépôt: 18749133.7
(22) Date de dépôt: 28.06.2018
(51) Int. Cl.: A61Q 11/00, A61C 19/06, A61K 9/00, A61K 8/19, A61M 35/00

(54) **DOSE UNITAIRE A USAGE UNIQUE POUR LE TRAITEMENT DE LA PARODONTOSE**
EINZELDOSEFORM ZUR EINMALIGEN NUTZUNG ZUR BEHANDLUNG VON PARODONTOSE
A SINGLE-USE UNIT DOSE FOR THE TREATMENT OF PERIODONTITIS

(30) Priorité: 05.07.2017 FR 1770710
(43) Date de publication de la demande: 13.05.2020
(73) Titulaire: 3DOM SAS, 63210 Olby (FR)
(72) Inventeur: DUHAMEL, François, 63460 Beauregard-Vendon (FR)
(74) Mandataire: Thurgood, Alexander John
(86) Numéro de dépôt international: PCT/IB2018/054811
(87) Numéro de publication internationale: WO 2019/008479

(56) Documents cités:
- EP-A1- 0 392 483
- DE-U1-202009 001 687
- US-A- 4 665 901
- US-A- 4 891 211
- US-A- 5 374 368
- US-A1- 2015 352 044

## Description

La présente invention concerne le domaine général du traitement de la parodontose. Les maladies parodontales sont des maladies infectieuses concernant le parodonte, c'est-à-dire le tissu qui soutient les dents et tout particulièrement la gencive, l'os alvéolaire, le ligament alvéolo-dentaire et le cément. La parodontose est essentiellement due à des bactéries qui se sont accumulées à la base de la dent et qui se multiplient. Cependant, la neutralisation de ces bactéries dans cette zone peut s'avérer difficile, car un écosystème bactérien est créé dans lequel se trouve la colonisation bactérienne à l'état simple, ainsi qu'un biofilm lui-même constitué de colonies de différentes bactéries recouvrant l'infection primaire et rendant les traitements chimiques classiques inopérants. Cette maladie fait que l'os alvéolaire autour des dents est détruit lentement, mais progressivement et par conséquence, cela conduit à une perte de la dent atteinte. Les études épidémiologiques réalisées sur le territoire national français évaluent à environ 16 millions d'adultes la population potentiellement concernée par cette pathologie dans la tranche d'age de 35 à 64 ans. Selon ces mêmes études, on estime à environ 800.000 à 1,000.000 individus qui seraient concernés par la forme sévère de cette pathologie sur le territoire national français.

Bien que des traitements relatifs à cette pathologie existent, ils sont à la fois longs, couteux et parfois douloureux. Par conséquent, on a proposé par le passé des traitements préventifs ou prophylactiques consistant à limiter les risques de contamination en particulier à la base de la dent, par le brossage régulier, éventuellement accompagné de l'application de de solutions comprenant des substances présentant une activité destructrices de la membrane cellulaire des bactéries, par exemple, des bains de bouche comportant des surfactants. Cependant, les bactéries responsables de cette attaque du parodonte sont de type Gram négatif et sont souvent protégées par un biofilm rendant très difficile tout traitement chimique.

Un autre traitement est également connu, qui consiste à préparer une pâte contenant du peroxyde d'hydrogène mélangé avec du bicarbonate de sodium. La pâte ainsi obtenue doit avoir une tenue et viscosité adaptée au brossage de la base de la dent. Elle est préparée par le patient atteint juste avant son utilisation en prenant par exemple, une cuillère à café de bicarbonate de sodium sous forme de poudre et en y ajoutant du peroxyde d'hydrogène en solution aqueuse stabilisée à une concentration de 5 volumes de peroxyde d'hydrogène et en mélangeant les deux pour en faire une pâte. L'aspect abrasif des particules de bicarbonate de sodium dans la pâte appliquée par brossage permet de rompre mécaniquement le biofilm et dégager les colonies bactériennes. Le peroxyde d'hydrogène, qui se dégrade en présence d'espèces protonées ou cationiques en présence d'eau dans la bouche pour donner de l'eau et de l'oxygène sous forme gazeuse, permet d'éliminer les bactéries Gram négatifs et anaérobies situées en dessous du biofilm. Cependant, ce traitement présente une faiblesse majeure. La réalisation de la préparation pâteuse correctement de manière quotidienne, voire pluri-quotidienne, s'avère rapidement trop difficile à réaliser et contraignant pour le patient. Cette problématique majeure est un véritable frein à l'observance du traitement et donc au traitement satisfaisant de cette pathologie.

Par ailleurs, le problème de la réactivité du peroxyde d'hydrogène est bien connu. On connaît donc dans l'art antérieur des formulations qui tentent de contourner la difficulté de la stabilisation du peroxyde d'hydrogène dans le temps, en combinant par exemple plusieurs substances présentées dans une formulation solide, pâteuse ou de gel, et qui vont réagir une fois exposées à l'eau pour produire du peroxyde d'hydrogène. Il s'agit notamment des sels alcalins ou alcalino-terreux de perhydrates, tels que le perborate ou le percarbonate.

Le brevet US5374368, par exemple, divulgue une composition sous forme liquide ou gel, capable de libérer du peroxyde d'hydrogène une fois exposée à l'eau, par exemple dans la bouche, ou appliquée sur une brosse à dents mouillée, et contenant du percarbonate de sodium. Le percarbonate de sodium, qui va réagir au contact de protons dans le milieu aqueux de la bouche pour former le peroxyde d'hydrogène, est stabilisée dans la composition sous forme de gel ou sous forme liquide, en le mélangeant avec du polyalkylène glycol, de la silice colloïdale, et du pyrophosphate de métaux alcalins, ainsi qu'avec un silicate de sodium ou de magnésium, ou d'un sulfate de magnésium et de l'eau.

Dans une approche semblable, le brevet américain US5424060 divulgue une composition de dentifrice, comprenant du bicarbonate de sodium en tant qu'agent abrasif, et du percarbonate de sodium pour libérer du peroxyde d'hydrogène lors de l'application de la composition dans la bouche, par exemple, lors du brossage des dents. La composition comporte de l'eau. Du propre aveu des auteurs de cette composition, celle-ci perd néanmoins de l'oxygène à raison de 4 % sur 21 jours et à 37,5°C.

Dans encore une autre approche, le brevet américain US4925655 présente une composition sous forme de mélange de poudres, le mélange étant soluble dans l'eau pour former une bain de bouche, et comprenant entre autres du peroxyde de calcium, du perborate de sodium et du bicarbonate de sodium. Il y est indiqué que le perborate de sodium peut être remplacé par du percarbonate de sodium.

Le brevet EP0279130 divulgue une composition sous forme de poudre pour le brossage des dents dans le traitement de la parodontose, comprenant au moins 40 % en poids d'un mélange de bicarbonate de sodium avec du percarbonate de sodium, composition qui libère, au contact de l'eau, entre 0,5 % et 5 % de peroxyde d'hydrogène actif, par rapport au poids total de la composition.

Malgré ces différentes tentatives de l'art antérieur, aucune solution n'a été trouvée à la difficulté du maintien dans le temps de la stabilité des composants d'un mélange comprenant du bicarbonate de sodium et du percarbonate de sodium. Le déposant a remarqué que toutes ces tentatives partaient de l'hypothèse que la composition demeurerait stable dans le temps, quelqu'en soit la nature de l'environnement de stockage ou les conditions ambiantes autour de la composition, même sous forme de mélange de poudres sèches. Par ailleurs, les compositions connues ont toujours été formulées en vue de gros conditionnements permettant plusieurs utilisations de la composition à la suite, ce qui a eu pour conséquence d'altérer les conditions d'humidité et donc de stabilité des mélanges de substances telles que le bicarbonate de sodium et le percarbonate de sodium.

Par conséquent, un objet de la présente invention est une dose unitaire à usage unique pour le traitement de la parodontose à base d'un mélange de poudres sèches, selon laquelle :
le mélange de poudres sèches comprend du bicarbonate de sodium et du percarbonate de sodium ; et
la dose unitaire se présente sous forme d'un conditionnement étanche du mélange de poudres sèches dans lequel ledit mélange est stocké sous atmosphère inerte.

En effet, aucune formulation de l'art antérieur ne divulgue de dose unitaire à usage unique conformément à l'invention, et dont le conditionnement est étanche et le mélange de poudres sèches est stocké dans ce conditionnement sous atmosphère inerte. Une telle présentation permet, entre autres, de disposer de la bonne dose de mélange de poudres à tout moment, sans que l'utilisateur soit obligé de mesurer, ou d'essayer de mesurer, et de doser le mélange lui-même. Par ailleurs, le conditionnement étant étanche et le mélange étant stocké sous atmosphère inerte, les problèmes de stabilité du mélange lié à son environnement disparaissent, ou sont a minima réduits à néant, ce qui fait que lorsque l'utilisateur vient se servir du produit, le mélange reste aussi actif que le jour où il a été formulé.

Pour les besoins de la présente invention, on entend par « mélange de poudres sèches » un mélange de poudres ne contenant sensiblement aucune eau ajoutée, ou eau ambiante, à savoir présentant un taux d'humidité résiduel inférieur ou égal à 60 %.

On entend généralement ici par atmosphère inerte une atmosphère environnante dans lequel le mélange est stocké qui n'interagit pas avec les composants du mélange, ne les déstabilise pas, ne les altère pas, ou ne les dégrade pas.

Selon un autre objet de la présente invention, l'atmosphère inerte comprend un gaz inerte. En effet, afin de stabiliser l'environnement de conditionnement du mélange, on préférera l'utilisation d'un gaz inerte ou d'un mélange de gaz inertes, pour créer l'atmosphère inerte. Ce gaz inerte pourra utilement être introduit dans le conditionnement avant sa fermeture, mais avant ou après introduction du mélange, selon des techniques connues dans l'industrie, par exemple en fermant le conditionnement de manière étanche sous flux de gaz inerte.

Selon encore un autre objet de la présente invention, l'atmosphère inerte est un gaz inerte choisi parmi l'argon et l'azote, ou un mélange de ceux-ci. On pourrait imaginer l'utilisation d'autres gaz inertes, en tant que de besoin.

Selon encore un autre objet de la présente invention, le conditionnement se présente sous forme de coupole étanche. Un tel conditionnement est utile par sa forme, puisqu'elle peut être utilisée par l'utilisateur à la manière d'une petite coupe, et parce qu'elle peut être empilée ou mise en carton pour former des unités de vente, voire reliées entre elles pour former des bandes de coupoles, telles les lentilles de contact, et donc aussi facilement transportables.

Selon encore un autre objet de la présente invention, la coupole comprend :
un corps de base muni d'une paroi inférieure, au moins une paroi latérale reliée à la paroi inférieure, et s'étendant depuis la paroi inférieure vers une extrémité supérieure, la paroi inférieure et la au moins une paroi latérale formant ensemble une coupole présentant une ouverture à l'extrémité supérieure ; et
une fermeture recouvrant l'ouverture de l'extrémité supérieure de manière à fermer celle-ci de manière étanche.

Selon une forme de coupole préférée, celle-ci présente une paroi inférieure fermée décrivant un cercle ou une partie d'un sphère, et la au moins une paroi latérale s'étend depuis la base en périphérie du cercle ou de partie de sphère formant ainsi une paroi périphérique latérale. Si la coupole ne comporte qu'une seule paroi latérale, celle-ci suivra en s'étendant vers le haut depuis la base, soit pour former une paroi droite, soit une paroi évasée et/ou tronconique, de manière à former une ouverture à l'extrémité supérieure de la paroi latérale. Bien entendu, on pourra imaginer d'autres formes de paroi de la base et de parois latérales correspondantes, par exemple, sensiblement rectangulaire ou carré ou polygonale pour la paroi de la base, avec des parois latérales correspondantes. L'ouverture à l'extrémité supérieure est recouverte d'une fermeture qui ferme l'ouverture de manière étanche.

La coupole peut être en toute matière adaptée, par exemple, en verre ou en matière plastique étanche, mais de préférence en matière plastique présentant une barrière étanche à l'entrée dans, ou à la sortie depuis le conditionnement, de gaz ou d'autres fluides tant que le conditionnement n'aura pas été ouvert. De telles matières convenables sont choisies dans le groupe consistant en le polypropylène basse densité, le polypropylène haute densité, le polyéthylène basse densité, le polyéthylène haute densité, le polyéthylène-téréphtalate, le chlorure de polyvinyle, et le copolymère d'éthylène et d'alcool vinylique, ou des combinaisons ou mélanges de ceux-ci.

Par exemple, et de préférence, la fermeture étanche est avantageusement un film, par exemple également en matière plastique étanche, avantageusement choisi dans le groupe consistant en le polypropylène basse densité, le polypropylène haute densité, le polyéthylène basse densité, le polyéthylène haute densité, le polyéthylène-téréphtalate, le chlorure de polyvinyle, et le copolymère d'éthylène et d'alcool vinylique, ou des combinaisons ou mélanges de ceux-ci. Généralement, le film présentera une épaisseur inférieure à l'épaisseur des parois de la base et latérale de la coupole. Il pourra être collé, par exemple sur le bord supérieur de la paroi latérale, par thermofusion, ou sinon avec un produit adhésif adéquat, de manière à former un joint étanche.

Selon un autre objet de la présente invention, la fermeture étanche est un film séparable ou déchirable à la main. Dans ce cas, le film séparable ou déchirable peut être pourvu avantageusement de lignes de déchirure facilitant sa séparation ou sa déchirure au niveau du bord supérieur de la paroi latérale, pour laisser la paroi latérale entière et permettre l'accès via l'ouverture au mélange de poudres.

Selon un autre objet de la présente invention, le conditionnement se présente sous forme d'un bâtonnet du mélange comprimé de poudres sèches, enrobé ou enveloppé de matière étanchéifiante. En effet, les déposants ont découvert qu'il était possible de conditionner le mélange de poudres sèches sous forme de bâtonnet, par exemple par compression, et l'envelopper ou l'enrober d'une couche étanche ou d'une enveloppe étanche, tout en prévoyant une atmosphère inerte autour du bâtonnet. L'enveloppe étanche peut également être en matière plastique, par exemple en film de matière plastique choisie dans le groupe consistant en le polypropylène basse densité, le polypropylène haute densité, le polyéthylène basse densité, le polyéthylène haute densité, le polyéthylène-téréphtalate, le chlorure de polyvinyle, et le copolymère d'éthylène et d'alcool vinylique, ou des combinaisons de ceux-ci. Elle peut également être pourvue de moyens permettant sa déchirure ou sa séparation du bâtonnet au moment de son utilisation. Notamment, et entre autres selon un autre objet de la présente invention, la matière étanchéifiante de la couche ou enveloppe étanche est séparable ou déchirable à la main, par exemple en tirant dessus le long d'une ou plusieurs lignes de déchirure pratiquées dans l'enveloppe.

Selon un autre objet de la présente invention, le conditionnement comporte un indicateur de volume de liquide à ajouter au mélange de poudres sèches lors de son utilisation. En effet, il est avantageux de prévoir un tel indicateur pour permettre à l'utilisateur de savoir avec précision combien de volume de liquide qu'il faudra ajouter au mélange pour préparer une pâte à brosser conformément au protocole de traitement de la parodontose.

Selon un autre objet de la présente invention, le liquide à ajouter au mélange de poudres sèches lors de son utilisation est de l'eau, de préférence de l'eau désionisée, mais il également s'agir de l'eau du robinet.

Selon encore un autre objet de la présente invention, le volume total du conditionnement étanche est compris entre environ 3 ml et environ 30 ml. Un tel volume a été déterminé comme particulièrement avantageux pour une dose unitaire prête à l'usage contenant à la fois le mélange de poudres et le liquide ajouté, notamment de l'eau, pour former une pâte à brosser correspondante. Selon un autre objet de la présente invention, le mélange de poudres sèches présente une distribution de tailles de particules dont 50 % au moins présente une taille de particules inférieure à 400 microns, de préférence inférieure à 200 microns.

Selon encore un autre objet de la présente invention, le mélange de poudres sèches présente une distribution de tailles de particules dont 70 % au moins présente une taille de particules inférieure à 400 microns, de préférence inférieure à 200 microns.

De préférence, la taille des particules médiane est comprise entre environ 74 microns et environ 210 microns, et plus préférentiellement encore entre environ 74 microns à environ 179 microns.

Selon un autre objet de la présente invention, dans le mélange de poudres sèches, le rapport pondéral de bicarbonate de sodium au percarbonate de sodium, par rapport au poids total du mélange, est d'environ 1 : 0,02. Le déposant a en effet déterminé qu'un tel rapport était très avantageux pour former la dose unitaire et assurer une efficacité optimale une fois le mélange transformé en pâte par ajout d'eau.

Enfin, selon encore un autre objet de la présente invention, le mélange de poudres sèches comprend du bicarbonate de sodium et du percarbonate de sodium dans le rapport pondéral suivant par rapport au poids total du mélange :
bicarbonate de sodium (NaHCO3) : 4,5 g à 5,5 g
percarbonate de sodium (Na2CO3)2.(H2O2)3 : 84,06 mg à 102,74 mg.

La présente invention sera décrite plus en détails ci-après, en se référant utilement aux figures accompagnant et faisant partie intégrante de la présente demande, dans lesquelles :
la Figure 1 est une représentation schématique en vue perspective d'une première forme de dose unitaire à usage unique selon l'invention ;
la Figure 2 est une représentation schématique en vue de section de la dose unitaire à usage unique de la Figure 1.

### EXEMPLE

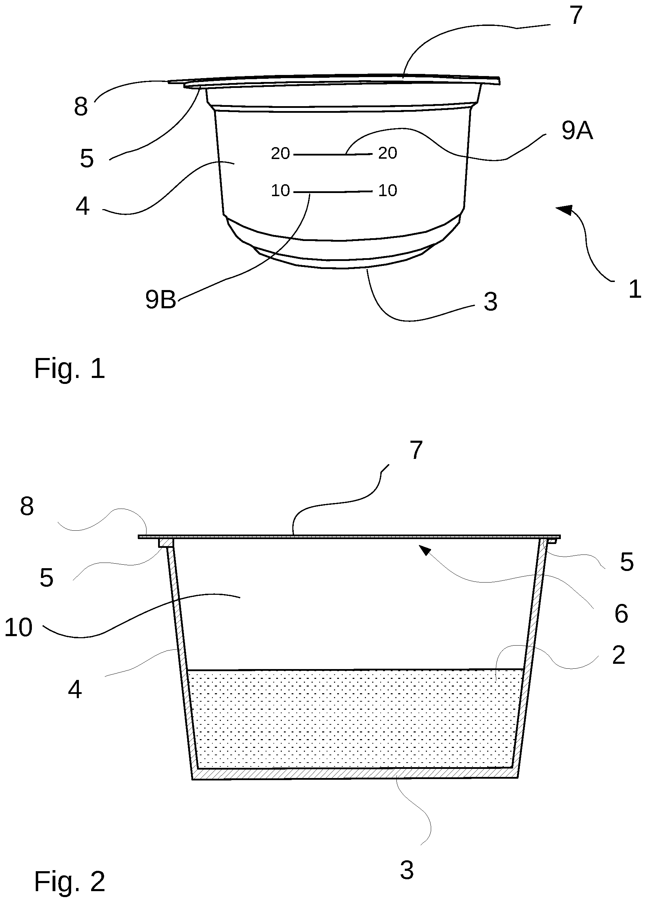

Les Figures 1 et 2 illustrent une première forme de dose unitaire à usage unique selon l'invention. Dans cette première forme, le conditionnement (1) d'un mélange (2) de poudres sèches comprenant du bicarbonate de sodium et du percarbonate de sodium se présente généralement sous la forme d'une coupole étanche (1). La matière plastique de la coupole (1) peut être transparente, de sorte que l'on puisse apercevoir le mélange de poudres à l'intérieur, ou comme illustré à la Figure1, opaque, voire translucide. La coupole est de préférence obtenue par injection ou soufflage dans un moule d'un polymère ou d'un mélange de polymères, ou par moulage d'un film multicouche comportant, par exemple, du polypropylène, du copolymère d'éthylène et d'alcool vinylique, et du polyéthylène (PP/EVOH/PE). Le corps de la coupole (1) étanche est formé d'une paroi de base inférieure (3), à partir de laquelle s'étend vers le haut une paroi latérale (4). La paroi latérale (4) présente un bord supérieur périphérique (5) en son extrémité supérieure, définissant une ouverture (6) dans la coupole (1), à travers laquelle le mélange de poudres sèches est introduit. Dans la Figure 1, l'ouverture est fermée par une fermeture (7), dont une partie est collée, par exemple, par thermocollage, ou par application d'un produit adhésif, sur le bord périphérique (5) de manière à recouvrir complètement et de manière étanche l'ouverture (6). La fermeture (7), qui peut être un film, ou une feuille, est avantageusement muni de moyens (8) permettant au moment de l'utilisation de la dose unitaire, de séparer la fermeture du corps de coupole et ainsi permettre à l'utilisateur d'ajouter un liquide, par exemple, de l'eau. Dans la Figure 1, les moyens (8) facilitant la séparation de le fermeture sont représentés par une languette (8) de préhension qui se projette à un point au-delà du bord périphérique supérieur (5) de la coupole (1). Lorsque l'utilisateur voudra se servir de la dose unitaire, il n'aura alors qu'à prendre la coupole (1) entre les doigts d'une main et puis soulever cette languette (8) avec les doigts de l'autre main et tirer dans la direction opposée de celle-ci pour séparer la fermeture (7) du bord supérieur de la coupole.

La Figure 2 représente la même forme de dose unitaire illustré en Figure 1, la vue étant cette fois-ci en coupe ou section transversale. Dans cette vue, on aperçoit que les parois de la base inférieure (3) et latérale (4) du corps de la coupole (1) présentent une épaisseur sensiblement égale et sont continues l'une avec l'autre, ce qui serait souvent le cas par exemple si la coupole est moulée par injection ou soufflage. Par contre, la fermeture (7) présente une épaisseur inférieure à celles des parois de base (3) et latérale (4). On distingue également dans cette vue deux indicateurs de volume (9A, 9B) de liquide à ajouter, prévu dans la paroi latérale (4), et dont la fonction est d'indiquer à un utilisateur au moment de l'utilisation de la dose unitaire, le volume de liquide, et en l'occurrence la plupart du temps de l'eau, à ajouter à la coupole (1). En effet, si l'utilisateur remplit la coupole (1) avec de l'eau jusqu'à un indicateur de volume (9A, 9B), il sera assuré d'obtenir non seulement la bonne consistance de pâte pour l'application du protocole de traitement de la parodontose, mais que cette pâte fonctionnera avec une efficacité optimale au niveau de la production de peroxyde d'hydrogène in situ, et ainsi la libération d'oxygène correspondante pour éliminer les bactéries. Plusieurs indicateurs (9A, 9B) de volume peuvent être prévus, comme illustré à la Figure 2, en fonction de la quantité de peroxyde d'hydrogène que l'on souhaite générer lors de l'ajout de l'eau. L'espace libre entre la fermeture (7) et le mélange de poudres sèches (2) est rempli d'atmosphère inerte (10), protégeant ainsi le mélange de toute dégradation, interaction ou de réaction intempestive ou involontaire avec l'environnement entourant le mélange. Avantageusement, cet atmosphère inerte est un gaz, tel que l'azote, ou un gaz dit « noble », tel que l'argon.

## Revendications

1. Dose unitaire à usage unique pour le traitement de la parodontose à base d'un mélange de poudres sèches, selon laquelle :
le mélange de poudres sèches comprend du bicarbonate de sodium et du percarbonate de sodium ; et
la dose unitaire se présente sous forme d'un conditionnement étanche du mélange de poudres sèches dans lequel ledit mélange est stocké sous atmosphère inerte.

2. Dose unitaire à usage unique pour le traitement de la parodontose selon la revendication 1, selon laquelle l'atmosphère inerte comprend un gaz inerte.

3. Dose unitaire à usage unique pour le traitement de la parodontose selon la revendication 1, selon laquelle l'atmosphère inerte est un gaz inerte choisi parmi l'argon et l'azote, ou un mélange de ceux-ci.

4. Dose unitaire à usage unique pour le traitement de la parodontose selon la revendication 1, selon laquelle le conditionnement se présente sous forme de coupole étanche.

5. Dose unitaire à usage unique pour le traitement de la parodontose selon la revendication 4, selon laquelle la coupole comprend :
un corps de base muni d'une paroi inférieure, au moins une paroi latérale reliée à la paroi inférieure, et s'étendant depuis la paroi inférieure vers une extrémité supérieure, la paroi inférieure et la au moins une paroi latérale formant ensemble une coupole présentant une ouverture à l'extrémité supérieure ; et
une fermeture recouvrant l'ouverture de l'extrémité supérieure de manière à fermer celle-ci de manière étanche.

6. Dose unitaire à usage unique pour le traitement de la parodontose selon la revendication 5, selon laquelle la fermeture étanche est un film séparable ou déchirable à la main.

7. Dose unitaire à usage unique pour le traitement de la parodontose selon la revendication 1, selon laquelle le conditionnement se présente sous forme d'un bâtonnet du mélange comprimé de poudres sèches, enrobé ou enveloppé de matière étanchéifiante.

8. Dose unitaire à usage unique pour le traitement de la parodontose selon la revendication 7, selon laquelle la matière étanchéifiante est séparable ou déchirable à la main.

9. Dose unitaire à usage unique pour le traitement de la parodontose selon la revendication 1, selon laquelle le conditionnement comporte un indicateur de volume de liquide à ajouter au mélange de poudres sèches lors de son utilisation.

10. Dose unitaire à usage unique pour le traitement de la parodontose selon la revendication 9, selon laquelle le liquide à ajouter au mélange de poudres sèches lors de son utilisation est de l'eau.

11. Dose unitaire à usage unique pour le traitement de la parodontose selon la revendication 1, selon laquelle le volume total du conditionnement étanche est compris entre environ 3 ml et environ 30 ml.

12. Dose unitaire à usage unique pour le traitement de la parodontose selon la revendication 1, selon laquelle le mélange de poudres sèches présente une distribution de tailles de particules dont 50 % au moins présente une taille de particules inférieure à 400 microns, de préférence inférieure à 200 microns.

13. Dose unitaire à usage unique pour le traitement de la parodontose selon la revendication 1, selon laquelle le mélange de poudres sèches présente une distribution de tailles de particules dont 70 % au moins présente une taille de particules inférieure à 400 microns, de préférence inférieure à 200 microns.

14. Dose unitaire à usage unique pour le traitement de la parodontose selon la revendication 1, selon laquelle dans le mélange de poudres sèches, le rapport pondéral de bicarbonate de sodium au percarbonate de sodium, par rapport au poids total du mélange, est d'environ 1 : 0,02.

15. Dose unitaire à usage unique pour le traitement de la parodontose selon la revendication 1, selon laquelle le mélange de poudres sèches comprend du bicarbonate de sodium et du percarbonate de sodium dans le rapport pondéral suivant par rapport au poids total du mélange :
bicarbonate de sodium (NaHCO3) : 4,5 g à 5,5 g
percarbonate de sodium (Na2CO3)2.(H2O2)3 : 84,06 mg à 102,74 mg.

## Patentansprüche

1. Einweg-Einheitsdosis zur Behandlung von Parodontose auf Basis von einem Trockenpulvergemisch, **dadurch gekennzeichnet, dass**:
das Trockenpulvergemisch Natriumbicarbonat und Natriumpercarbonat umfasst; und
die Einheitsdosis in Form einer versiegelten Verpackung des Trockenpulvergemisches vorliegt, in der das Gemisch unter einer inerten Atmosphäre gelagert wird.

2. Einweg-Einheitsdosis zur Behandlung von Parodontose nach Anspruch 1, wobei die inerte Atmosphäre ein Inertgas umfasst.

3. Einweg-Einheitsdosis zur Behandlung von Parodontose nach Anspruch 1, wobei die inerte Atmosphäre ein Inertgas ist, ausgewählt aus Argon und Stickstoff, oder deren Mischung.

4. Einweg-Einheitsdosis zur Behandlung von Parodontose nach Anspruch 1, wobei die Verpackung in Form einer versiegelten Kuppel vorliegt.

5. Einweg-Einheitsdosis zur Behandlung von Parodontose nach Anspruch 4, wobei die Kuppel:
einen Grundkörper mit einer Bodenwand, mindestens einer mit der Bodenwand verbundenen Seitenwand, die sich von der Bodenwand zu einem oberen Ende erstreckt, wobei die Bodenwand und die mindestens eine Seitenwand zusammen eine Kuppel mit einer Öffnung am oberen Ende bilden; und
einen Verschluss, der die Öffnung des oberen Endes abdeckt, um sie zu versiegeln, umfasst.

6. Einweg-Einheitsdosis zur Behandlung von Parodontose nach Anspruch 5, wobei der Verschluss eine von Hand abtrennbare oder abreißbare Folie ist.

7. Einweg-Einheitsdosis zur Behandlung von Parodontose nach Anspruch 1, wobei die Verpackung in Form eines Sticks des komprimierten Trockenpulvergemisches vorliegt, der mit Versiegelungsmaterial beschichtet oder umwickelt ist.

8. Einweg-Einheitsdosis zur Behandlung von Parodontose nach Anspruch 7, wobei das Versiegelungsmaterial von Hand abtrennbar oder abreißbar ist.

9. Einweg-Einheitsdosis zur Behandlung von Parodontose nach Anspruch 1, wobei die Verpackung eine Anzeige für die Flüssigkeitsmenge enthält, die dem Trockenpulvergemisch bei Gebrauch zugesetzt werden muss.

10. Einweg-Einheitsdosis zur Behandlung von Parodontose nach Anspruch 9, wobei die Flüssigkeit, die dem Trockenpulvergemisch bei Gebrauch zugesetzt wird, Wasser ist.

11. Einweg-Einheitsdosis zur Behandlung von Parodontose nach Anspruch 1, wobei das Gesamtvolumen der versiegelten Packung zwischen etwa 3 ml und etwa 30 ml liegt.

12. Einweg-Einheitsdosis zur Behandlung von Parodontose nach Anspruch 1, wobei das Trockenpulvergemisch eine Partikelgrößenverteilung aufweist, von der mindestens 50 % eine Partikelgröße von weniger als 400 Mikrometer, vorzugsweise weniger als 200 Mikrometer, aufweisen.

13. Einweg-Einheitsdosis zur Behandlung von Parodontose nach Anspruch 1, wobei das Trockenpulvergemisch eine Partikelgrößenverteilung aufweist, von der mindestens 70% eine Partikelgröße von weniger als 400 Mikrometer, vorzugsweise weniger als 200 Mikrometer, aufweisen.

14. Einweg-Einheitsdosis zur Behandlung von Parodontose nach Anspruch 1, wobei im Trockenpulvergemisch, das Gewichtsverhältnis von Natriumbicarbonat zu Natriumpercarbonat, bezogen auf das Gesamtgewicht der Mischung, etwa 1:0,02 beträgt.

15. Einweg-Einheitsdosis zur Behandlung von Parodontose nach Anspruch 1, wobei das Trockenpulvergemisch Natriumbicarbonat und Natriumpercarbonat im folgenden Gewichtsverhältnis, bezogen auf das Gesamtgewicht der Mischung, enthält:
Natriumbicarbonat (NaHCO3) : 4,5 g bis 5,5 g
Natriumpercarbonat (Na2CO3)2.(H2O2)3 : 84,06 mg bis 102,74 mg.

## Claims

1. Single use unit dose for the treatment of periodontosis based on a mixture of dry powders, wherein:
the mixture of dry powders comprises sodium bicarbonate and sodium percarbonate; and
the unit dose is provided as a sealed package containing the mixture of dry powders, said mixture being stored in an inert atmosphere.

2. Single use unit dose for the treatment of periodontosis according to claim 1, wherein the inert atmosphere comprises an inert gas.

3. Single use unit dose for the treatment of periodontosis according to claim 1, wherein the inert atmosphere is an inert gas chosen from argon and nitrogen, or a mixture thereof.

4. Single use unit dose for the treatment of periodontosis according to claim 1, wherein the package is in the shape of a sealed cupola.

5. Single use unit dose for the treatment of periodontosis according to claim 4, wherein the cupola comprises:
a base body having a lower wall, at least one side wall connected to the lower wall, and extending from the lower wall towards an upper end, the lower wall and the at least one side wall forming together a cupola having an opening at the upper end; and
a closure covering the opening at the upper end to sealingly close the opening.

6. Single use unit dose for the treatment of periodontosis according to claim 5, wherein the sealed closure is a film that is detachable or tearable by hand.

7. Single use unit dose for the treatment of periodontosis according to claim 1, wherein the package is in the shape of a stick of compressed mixture of the dry powders, coated or covered with a sealing material.

8. Single use unit dose for the treatment of periodontosis according to claim 7, wherein the sealing material is detachable or tearable by hand.

9. Single use unit dose for the treatment of periodontosis according to claim 1, wherein the package comprises a liquid volume indicator for a liquid to be added to the mixture of dry powders when used.

10. Single use unit dose for the treatment of periodontosis according to claim 9, wherein the liquid to be added to the mixture of dry powders when used is water.

11. Single use unit dose for the treatment of periodontosis according to claim 1, wherein the total volume of the sealed package is comprised between about 3 ml and about 30 ml.

12. Single use unit dose for the treatment of periodontosis according to claim 1, wherein the mixture of dry powders has a particle size distribution in which at least 50% have a particle size less than 400 microns, preferably less than 200 microns.

13. Single use unit dose for the treatment of periodontosis according to claim 1, wherein the mixture of dry powders has a particle size distribution in which at least 70% has a particle size less than 400 microns, preferably less than 200 microns.

14. Single use unit dose for the treatment of periodontosis according to claim 1, wherein in the mixture of dry powders, the weight ratio of sodium bicarbonate to sodium percarbonate, per total weight of the mixture, is about 1 : 0.02.

15. Single use unit dose for the treatment of periodontosis according to claim 1, wherein the mixture of dry powders comprises sodium bicarbonate and sodium percarbonate in the following weight ratio, per total weight of the mixture:
sodium bicarbonate (NaHCO₃) : 4.5 g to 5.5 g
sodium percarbonate (Na₂CO₃)₂. (H₂O₂)₃ : 84.06 mg to 102.74 mg.
